# EUROPEAN PATENT SPECIFICATION

(11) **EP 2 516 448 B1**
(45) Date of publication and mention of the grant of the patent: **15.04.2015**
(21) Application number: 10795485.1
(22) Date of filing: 03.12.2010
(51) Int. Cl.: C07D 501/22, A61K 31/546

(54) **AMINE SALTS OF CEFDINIR**
AMINSALZE VON CEFDINIR
SELS AMINE DE CEFDINIR

(30) Priority: 25.12.2009 TR 200909784
(43) Date of publication of application: 31.10.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000239
(87) International publication number: WO 2011/078819

(56) References cited:
- WO-A1-98/45299
- WO-A1-2006/053625

## Description

Present invention relates to pharmaceutically acceptable salts of cefdinir, specifically to primary, secondary and tertiary amine salts of cefdinir, preparation methods and pharmaceutical compositions comprising them.

Cefdinir molecule which is shown with Formula I was first disclosed in the patent numbered BE897864 and its chemical name is (6R,7R)-7-[[(2Z)-(2-amino-4-thiazolyl)(hydroxyimino)acetyl] amino]-3-ethenyl-8-oxo-5-thia-1-azabicyclo[4.2.0]oct-2-en-2-carboxylic acid. This molecule which is a third generation cephalosporin, is indicated for the treatment of several illnesses caused by gram positive and gram negative bacteria.

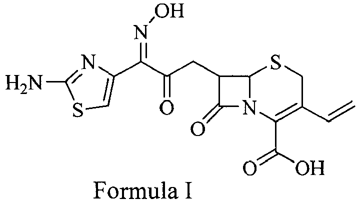

Although in vitro tests prove that cefdinir is a highly potent antibiotic, the bioavailability of the finished product is less than expected and manufacturers experience problems while developing formulations due to its poor solubility in water, methanol, ethanol, acetone and many other organic solvents.

Subject matter of the present invention is related to the development of water soluble derivatives of cefdinir without losing its antibiotic effect and to prepare pharmaceutical dosage forms comprising these derivatives.

### Detailed description of the invention

Present invention relates to organic amine salts of cefdinir, wherein said organic amines comprise at least one hydroxy group and relates to pharmaceutical dosage forms comprising said cefdinir salts.

The patent numbered EP1812449 discloses ammonium salt of cefdinir which can be used for the preparation cefdinir monohydrate. Another patent EP1546155, discloses ammonium and organic amine salts of cefdinir and use of these molecules as intermediates for synthesis of cefdinir.

As seen from these examples, prior art examples generally deal with the methods related to synthesis of cefdinir instead of its solubility problem.

Accordingly, present invention relates to the salts shown with the general Formula II.

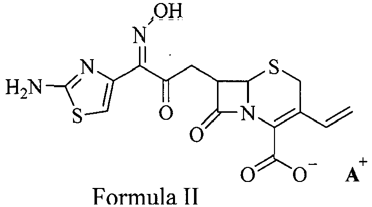

The group shown with "A" in Formula II is selected from ethanolamine, isopropanolamine, 1-deoxy-1-methylamino-sorbitol, 1-deoxy-1methylamino-D-glucitol, tris(hydroxymethyl)aminomethane, N-(tri(hydroxymethyl)methyl)glycine, N,N-Bis(2-hydroxyethylglycine), 2-methyl aminophenol, (2S,4R)-4-Hydroxy proline, thiamine.

In another aspect, one of the preferred molecules according to the present invention is the cefdinir salt shown with Formula III.

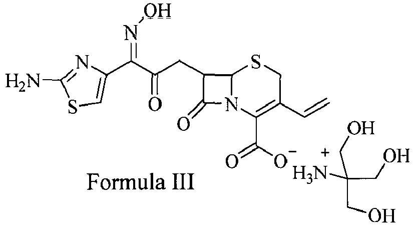

In another aspect, one of the preferred molecules according to the present invention is the cefdinir salt shown with Formula IV.

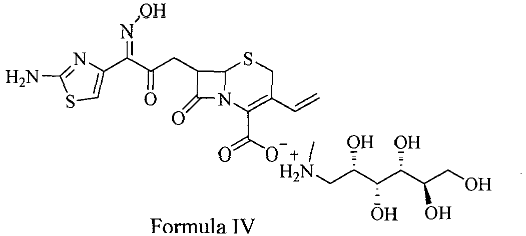

Inventors have found that cefdinir salts of the present invention have better water solubility compared to cefdinir in free base form and amine salts of cefdinir that do not comprise a hydroxy group which are known from the prior art.

In general, salts shown with Formula II are obtained with conventional techniques known from the prior art.

In another aspect present invention relates to use of the amine salts of cefdinir according to the present invention in solid and liquid dosage forms.

In another aspect present invention relates to use of the amine salts of cefdinir according to the present invention in dosage forms suitable for oral, buccal, sublingual application.

In another aspect present invention relates to use of the amine salts of cefdinir according to the present invention in pharmaceutical formulations in the form of film tablets, extended release tablets, modified release tablets, chewable tablets, effervescent tablets, effervescent granules, water dispersible tablet, water dispersible granules.

Amine salts of the present invention that is used in said pharmaceutical compositions can be in amorphous or crystal form.

In another aspect present invention relates to use of amine salts of cefdinir according to present invention in amounts equivalent to 1-4000 mg cefdinir.

In another aspect present invention relates to use of amine salts of cefdinir according to present invention and pharmaceutical compositions comprising them for the treatment of infections caused by gram positive and gram negative bacteria.

Molecules according to the present invention can be prepared according to but not limited with the examples given below.

### Example 1: Preparation of Tris(hydroxymethyl)aminomethane salt of cefdinir

39.5 g of cefdinir (0.1 mol) and 18.7 g tris(hydroxymethyl)aminomethane (0.15 mol) is stirred in a mixture of 100 mL ethanol and 50 mL deionized water at a temperature of 25-35 °C. The reaction mixture is then cooled to the room temperature and the formed precipitate is separated and recrystallized.

### Example 2: Preparation of 1-Deoxy-1-methylamino-sorbitol salt of cefdinir

39.5 g cefdinir (0.1 mol) and 58.6 g 1-Deoxy-1-methylamino-sorbitol (0.3 mol)is stirred in 100 mL of methanol at a temperature of 40 °C. The reaction mixture is cooled in a cooler and the precipitate that forms is filtrated right away and purified by washing with cold methanol.

In all stages of the preparation of the molecules of invention; all the known polar, non-polar, protic and aprotic organic solvents and water, all the known purification methods such as extraction, recrystallization with solvent/anti-solvent, recrystallization with activated carbon, chromatographic techniques, distillation and filtration can be used.

In another aspect, molecules of invention can form during the manufacture or use of a pharmaceutical composition comprising cefdinir and an amine comprising more than one hydroxy group.

Solvent that can be used for the preparation and purification of the molecules of invention can be choson from but not limited with a group comprising; water, ethanol, methanol, isopropanol, dimethylformamide, dimethylsulfoxide, methylenechloride, tetrahydrofuran, toluene, acetonitrile, hexane, heptane, diethylether, benzene, ethyl acetate, acetone, t-butyl alcohol, t-butyl methyl ether, chloroform, cyclohexane, 1,2-dichloroethane, 1,2-dimethoxyethane, dioxane, ethyl methyl ketone, ethylene glycol, 2-propanol, pyridine, triethylamine.

## Claims

1. Salts shown with Formula II; 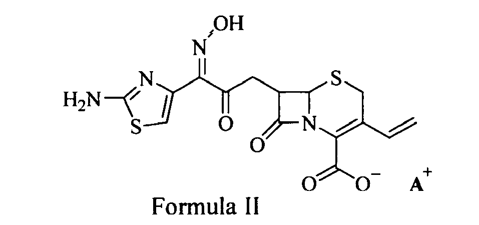 wherein A is selected from ethanolamine, 1-deoxy-1-methylamino-sorbitol, isopropanolamine, 1-deoxy-1-methylamino-D-glucitol- (2S,4R)-4-Hydroxy proline, tris(hydroxymethyl)aminomethane, N-(Tri(hydroxymethyl)methyl)glycine, thiamine, 2-methyl-aminophenol, N,N-Bis(2-hydroxyethyl)glycine.

2. A salt according to claim 1, wherein said salt is used in solid and liquid pharmaceutical dosage forms.

3. A solid dosage form according to claim 2 which is suitable for oral, buccal, sublingual application.

4. A dosage form according to claim 2 and 3 which is in the form of film coated tablets, extended release tablets, modified release tablets, chewable tablets, effervescent tablets, effervescent granules, suspensions, water dispersible tablets, water dispersible granules.

5. A pharmaceutical composition according to claim 2 and 4 wherein said composition comprises the salts according to claim 1 in an amount equivalent to 1-4000 mg of cefdinir.

6. A pharmaceutical composition according to any of the preceding claims for use in treatment of infections caused by gram positive and gram negative bacteria.

7. A salt according to claim 1 which is in amorphous or crystal form.

8. A process for preperation of the salts according to claim 1 comprising the steps of;
a) Dissolving cefdinir and an organic amine selected from the group comprising ethanolamine, isopropanolamine, 1-deoxy-1-methylamino-sorbitol, 1-deoxy-1-methylamino-D-glucitol, (2S,4R)-4-hydroxyproline, tris(hydroxymethyl)aminomethane, N-(tri(hydroxymethyl)methyl)glycine, thiamine, 2-methyl aminophenol, N,N-Bis(2-hydroxyethyl)glycine in a suitable solvent and stirring said solution at a temperature in the range of 0-100 °C
b) Separation of the product that precipitates upon cooling of the reaction mixture, and optionally
c) Purification of the obtained product.

9. A process according to claim 8 wherein the solvent used in said process can be selected from a group comprising polar, non-polar, aprotic, protic organic solvents, water and/or a combination of these, preferably wherein the solvent used in said process can be selected from a group comprising water, ethanol, methanol, isopropanol, dimethylformamide, dimethylsulfoxide, methylenechloride, tetrahydrofuran, toluene, acetonitrile, hexane, heptane, diethylether, benzene, ethyl acetate, acetone, t-butyl alcohol, t-butyl methyl ether, chloroform, cyclohexane, 1,2-dichloroethane, 1,2-dimethoxyethane, dioxane, methyl ethyl ketone, ethylene glycol, 2-propanol, pyridine, triethylamine.

10. A process according to claim 8 wherein the reaction is carried out at a temperature of 0-100 °C, preferably at e temperature of 10-70 °C, more preferably at a temperature of 20 - 60 °C.

11. A process according to claim 8, wherein the product can be separated from the reaction mixture by any one of the methods comprising extraction, filtration and/or evaporation of the solvent.

12. A process according to claim 8 wherein said product can be purified by use of the techniques such as extraction, recrystallization by solvent, recrystallization by activated charcoal, all chromatographic techniques, distillation, filtration alone or in a combined form.

13. Cefdinir salt shown with Formula III

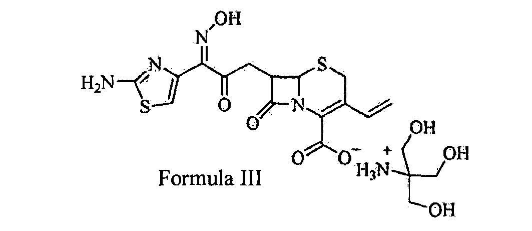

14. Cefdinir salt shown with Formula IV

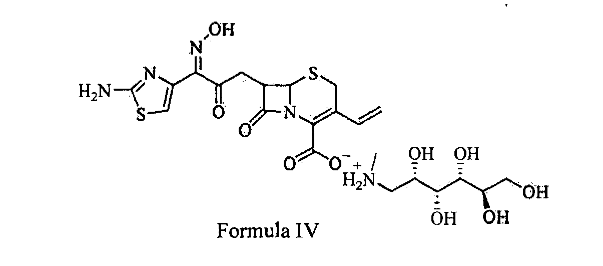

15. Organic amine salts according to any of the preceding claims are **characterized in that** said organic amines salts are formed during the manufacture or use of a pharmaceutical composition comprising cefdinir and an amine having more than one hydroxy group.

## Patentansprüche

1. Salze, die mit der Formel II dargestellt sind; 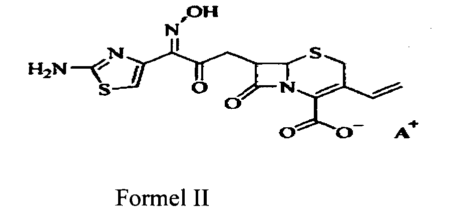 wobei A von Ethanolamin, 1-deoxy-1-methylamino-sorbitol, Isopropanolamin, 1-deoxy-1-methylamino-D-glucitol- (2S, 4R) -4-Hydroxyproline, Tris (hydroxymethyl) aminomethan, N-(Tri (hydroxymethyl) methyl)glycin, Thiamin, 2-Methyl-aminophenol, N, N-Bis (2- hydroxyethyl) glycin ausgewählt wird.

2. Ein Salz gemäß Anspruch 1, wobei das erwähnte Salz in festen und flüssigen pharmazeutischen Arzneiformen verwendet wird.

3. Eine feste Dosierungsform gemäß Anspruch 2, die sich für orale, bukkale, sublinguale Anwendung geeignet ist.

4. Eine Dosierungsform gemäß Anspruch 2 und 3, die in Form von Filmtabletten, erweiterten Release-Tabletten, modifizierten Freisetzungstabletten , Kautabletten , Brausetabletten , Brausegranulaten, Suspensionen, wasserdispergierbaren Tabletten, wasserdispergierbaren Granulaten ist.

5. Eine pharmazeutische Zusammensetzung gemäß Anspruch 2 und 4, wobei die erwähnte Zusammensetzung die Salze gemäß Anspruch 1 in einer Menge, die 1-4000 mg von Cefdinir entspricht, beinhaltet.

6. Eine pharmazeutische Zusammensetzung gemäß einem der vorangehenden Ansprüche zur Verwendung bei der Behandlung von Infektionen, die durch gram-positive und gramnegative Bakterien verursacht werden.

7. Salz gemäß Anspruch 1, welches in amorpher oder kristalliner Form ist.

8. Ein Verfahren zur Vorbereitung der Salze gemäß Anspruch 1, wobei das die folgenden Schritte umfasst;
a) Auflösen von Cefdinir und ein organisches Amin, aus der Gruppe bestehend aus Ethanolamin, Isopropanolamin, 1-deoxy-1-methylamino-sorbit, 1-Deoxy-1- methylamino-D-glucitol, (2S, 4R) -4-Hydroxyprolin, Tris (hydroxymethyl) -aminomethan, N- (Tri (hydroxymethyl) methyl) glycin, Thiamin, 2-Methyl-Aminophenols, N, N-Bis (2-Hydroxyethyl) glycin in einem geeigneten Lösungsmittel ausgewählt wird und Rühren der erwähnte Lösung bei einer Temperatur im Bereich von 0-100°C.
b) Abtrennung des Produktes, das beim Abkühlen der Reaktionsmischung und gegebenenfalls ausfällt
c) Die Reinigung des erhaltenen Produkts.

9. Ein Verfahren gemäß Anspruch 8, wobei das in diesem Verfahren verwendete Lösungsmittel aus einer Gruppe ausgewählt werden kann, die polare, nicht-polare, aprotische, protische organische Lösungsmittel, Wasser und/oder eine Kombination davon, wobei vorzugsweise das in diesem Verfahren verwendete Lösungsmittel aus einer Gruppe ausgewählt werden kann, die Wasser, Ethanol, Methanol, Isopropanol, Dimethylformamid, Dimethylsulfoxid, Methylenchlorid, Tetrahydrofuran, Toluol, Acetonitril, Hexan, Heptan, Diethylether, Benzol, Ethylacetat, Aceton, t-Butylalkohol, t-Butylmethylether , Chloroform, Cyclohexan, 1,2-Dichlorethan, 1,2-Dimethoxyethan, Dioxan, Methylethylketon, Ethylenglykol, 2-Propanol, Pyridin, Triethylamin umfasst.

10. Ein Verfahren gemäß Anspruch 8, wobei die Reaktion bei einer Temperatur von 0-100°C, vorzugsweise bei einer Temperatur von 10-70°C und besonders bevorzugt bei einer Temperatur von 20 - 60°C durchgeführt wird.

11. Ein Verfahren gemäß Anspruch 8, wobei das Produkt durch irgendeines der Verfahren, das die Extraktion, Filtration und/oder Verdampfen des Lösungsmittels beinhaltet, von dem Reaktionsgemisch abgetrennt werden kann.

12. Ein Verfahren gemäß Anspruch 8, wobei das erwähnte Produkt durch die Verwendung der Techniken wie Extraktion, Rekristallisation durch Lösungsmittel, Umkristallisation durch Aktivkohle , alle chromatographische Methoden, Destillation, Filtration allein oder in einer kombinierten Form gereinigt werden kann.

13. Cefdinir Salz, das mit der Formel III dargestellt wird

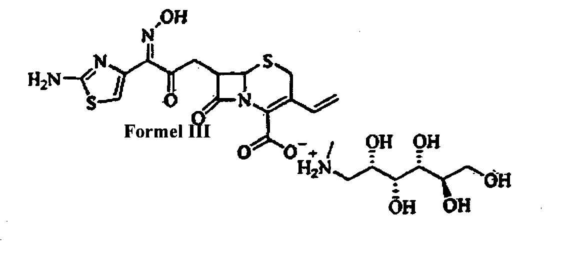

14. Cefdinir Salz, das mit der Formel IV dargestellt wird

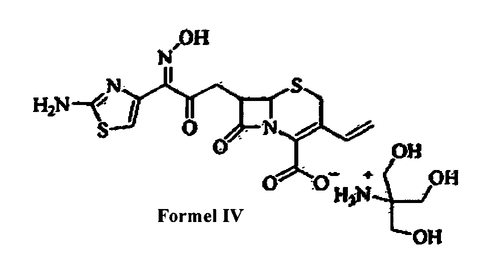

15. Organische Aminsalze gemäß einem der vorangehenden Ansprüche, **dadurch gekennzeichnet, dass** die erwähnte organischen Aminsalze während der Herstellung oder Verwendung einer pharmazeutischen Zusammensetzung, die Cefdinir und ein Amin mit mehr als einer Hydroxygruppe beinhaltet, gebildet werden.

## Revendications

1. Les sels indiqués avec la formule II; 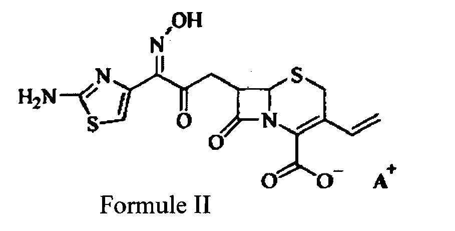 dans laquelle, A est choisi parmi éthanolamine, 1-désoxy-1-méthylamino-sorbitol, isopropanolamine, 1 -désoxy-1 -méthylamino-D-glucitol- (2S,4R)-4-Hydroxy proline, tris(hydroxyméthyl)aminométhane, N-(Tri(hydroxyméthyl)méthyl)glycine, thiamine, 2-méthyl-aminophénol, N,N-Bis(2-hydroxyéthyl)glycine.

2. Un sel selon la revendication 1, dans lequel, ledit sel est utilisé sous la forme des dosages pharmaceutiques solides et liquides.

3. Une forme de dosage pharmaceutique solide selon la revendication 2 apte à une application oral, buccal, sublingual.

4. Une forme de dosage selon les revendications 2 et 3 qui est sous la forme d'un comprimé pelliculé, un comprimé à libération prolongée, un comprimé à libération modifiée, un comprimé à croquer, un comprimé effervescent, un granule effervescent, une suspension, un comprimé dispersible dans l'eau, un granule dispersible dans l'eau.

5. Une composition pharmaceutique selon les revendications 2 et 4, dans laquelle, ladite composition comprend les sels selon la revendication 1 dans un montant équivalent à 1-4000 mg du cefdinir.

6. Une composition pharmaceutique selon quelconque des revendications précédents pour utiliser dans le traitement des infections causées par les bactéries grams positives et négatives.

7. Un sel selon la revendication 1 sous une forme amorphe et cristalline.

8. Un procède de préparation des sels selon la revendication 1, comprenant les étapes suivantes:
a) Dissoudre le cefdinir et une amine organique choisie parmi le group comprenant éthanolamine, isopropanolamine, 1-désoxy-1-méthylamino-sorbitol, 1-désoxy-1-méthylamino-D-glucitol, (2S,4R)-4-Hydroxyproline, tris(hydroxyméthyl)aminométhane, N-(tri(hydroxyméthyl)méthyl)glycine, thiamine, 2-méthyl aminophénol, N,N-Bis(2-hydroxyethyl)glycine dans un solvant approprié et mélanger ladite solution à une température dans la plage de 0-100 °C.
b) Séparation du produit qui précipite par refroidissement du mélange réactionnel, et éventuellement
c) Purification du produit obtenu.

9. Un procède selon la revendication 8, dans lequel le solvant utilisé dans ledit procède peut être choisi dans un group comprenant les solvants organiques polaires, non-polaires, aprotiques, protiques, l'eau et/ou une combinaison de ceux-ci, de préférence dans laquelle le solvant utilisé dans ledit procède peut être choisi dans un group comprenant l'eau, éthanol, méthanol, isopropanol, diméthylformamide, diméthylsulfoxyde, chlorure de méthylène, tétrahydrofurane, toluène, acétonitrile, hexane, heptane, éther diéthylique, benzène, acétate d'éthyle, acétone, alcool butylique tertiaire, méthyl-tertio-butyl-éther, chloroforme, cyclohexane, 1,2-dichloroéthane, 1,2-diméthoxyéthane, dioxane, méthyléthylcétone, éthylèneglycol, 2-propanol, pyridine, triéthylamine.

10. Un procède selon la revendication 8 dans lequel la réaction est réalisée à une température de 0-100°C, de préférence à une température de 10-70°C, et de manière plus préféré à une température de 20-60°C.

11. Un procède selon la revendication 8, dans lequel le produit peut être séparé depuis le mélange réactionnel par quelconque des méthodes comprenant extraction, filtration et/ou évaporation du solvant.

12. Un procède selon la revendication 8, dans lequel ledit produit peut être purifié par utilisation des techniques telles que l'extraction, recristallisation par solvant, recristallisation par charbon actif, tous les techniques chromatographique, distillation, filtration seulement ou dans une forme combinée.

13. Le sel de cefdinir indiqué avec la Formule III

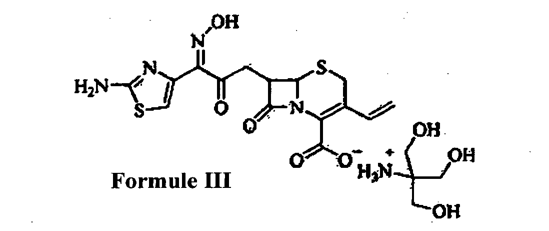

14. Le sel de cefdinir indiqué avec la Formule IV

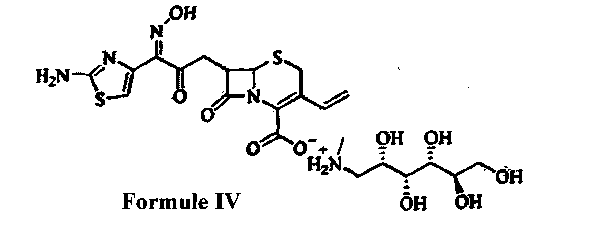

15. Les sels d'amines organiques selon quelconques des revendications précédents, **caractérisé en ce que** lesdites amines organiques sont formées pendent la fabrication ou l'utilisation d'une composition pharmaceutique comprenant du cefdinir et une amine ayant plus d'un group d'hydroxy.
